# EUROPEAN PATENT APPLICATION

(11) **EP 2 783 698 A1**
(43) Date of publication of application: **01.10.2014**
(21) Application number: 14169349.9
(22) Date of filing: 18.08.2008
(51) Int. Cl.: A61K 38/19, G01N 33/53, A61P 3/04, G01N 33/68

(54) **Agents and methods for modulating macrophage inhibitory cytokine (MIC-1) activity**

(30) Priority: 16.08.2007 AU 2007904412 P
(62) Divisional of application: 08782955.2
(71) Applicant: ST VINCENT'S HOSPITAL SYDNEY LIMITED, Sydney, NSW 2010 (AU); Garvan Institute of Medical Research, Darlinghurst, NSW 2010 (AU)
(72) Inventor: Breit, Samuel Norbert, Gordon, New South Wales 2072 (AU); Brown, David Alexander, Lane Cove, New South Wales 2066 (AU); Herzog, Herbert, Bondi, New South Wales 2026 (AU); Lin, Shu, Lidcombe, New South Wales 2141 (AU)
(74) Representative: Harding, Charles Thomas

(57) **Abstract**

The invention relates to a method and novel types of agents for modulating appetite and/or body weight in a subject. Moreover, the invention relates to a method of screening for agents which interact and modulate the activity of the receptor complex for macrophage inhibitory cytokine-1 (MIC-1).

## Description

### FIELD OF THE INVENTION

The invention relates to a method and novel types of agents for modulating appetite and/or body weight in a subject. Moreover, the invention relates to a method of screening for agents which interact and modulate the activity of the receptor complex for macrophage inhibitory cytokine-1 (MIC-1).

### INCORPORATION BY REFERENCE

This patent application claims priority from:
- AU 2007904412 titled "Agents and methods for modulating macrophage inhibitory cytokine-1 (MIC-1) activity" filed on 16 August 2007.

The entire content of this application is hereby incorporated by reference.

Also, the following patent specifications are referred to in the following description:
- International Patent Application No PCT/AU2005/000525 (WO 2005/099746); and
- United States Patent No 5,225,539.

The entire content of each of these two patent specifications is hereby incorporated by reference.

### BACKGROUND OF THE INVENTION

Weight and appetite control are complex, incompletely characterised processes. Their dysregulation may lead to obesity, a significant world-wide public health issue. At the other extreme, anorexia/cachexia is most commonly due to late stage cancer, where it is believed that tumour or stromal cell derived molecules disturb the stringent control of appetite and weight, leading to wasting, debility and often death¹⁻⁶. Whilst several cytokines have been implicated in the pathogenesis of anorexia/cachexia, interleukin-6 (IL-6) has been considered the likeliest aetiological agent. However, to date there have been no human clinical trials of monoclonal antibodies to IL-6 demonstrating efficacy in treating cancer anorexia/cachexia.

MIC-1, also called GDF-15, PLAB, PDF and NAG-1, is a divergent member of the TGF-β superfamily that is not expressed under basal conditions but may be induced by inflammation, injury or the development of malignancy⁷⁻¹³. MIC-1 is overexpressed by many cancers, including those of the prostate, colon, pancreas and breast¹⁴. In patients with advanced cancer, this results in a marked increase in serum MIC-1 levels, which can rise by 10-100 fold from a mean of 450 pg/ml to levels of 5-50 ng/ml or higher. Individual tumours may vary in the amount of MIC-1 they secrete because of both individual differences in expression level and variation in processing of the mature MIC-1 by pro-convertases ¹¹. The propeptide of MIC-1 binds strongly to extracellular matrix (ECM) resulting in the formation of ECM stores of MIC-1, that are demonstrable both *in vitro* and *in vivo.* Only the processed form of MIC-1, lacking the matrix binding propeptide, diffuses into the circulation. Thus, the propeptide regulates the balance between ECM stores and circulating blood levels of MIC-1 *in vivo*¹¹.

The present applicant has previously described in International Patent Application No PCT/AU2005/000525 (WO 2005/099746), methods and agents for modulating appetite and/or body weight in a subject involving the modulation of MIC-1 activity. A particular type of agent described therein are anti-MIC-1 antibodies which cause inhibition of MIC-1 activity and are capable of causing an increase in appetite and/or body weight in a subject. Provided hereinafter is further evidence that tumours overexpressing MIC-1 induce anorexia/cachexia which can be reversed by administration of antibodies to MIC-1 and induced by injection of recombinant MIC-1. Further, the mechanism of MIC-1 action has been investigated, leading to characterisation of the MIC-1 receptor complex and the potential central regulators which effect modulation of appetite and energy homeostasis.

Accordingly, the present applicant has identified, *inter alia,* a method and novel types of agents for modulating appetite and/or body weight.

### SUMMARY OF THE INVENTION

The present invention relates to the identification of novel types of agents for modulating the activity of macrophage inhibitory cytokine-1 (MIC-1), wherein the agents modulate the activity of a MIC-1 receptor complex comprising the RII receptor for transforming growth factor-β (TGF-β). By modulating the activity of MIC-1, the agents are capable of modulating appetite and/or body weight of a subject.

Thus, in a first aspect, the present invention provides a method of modulating the activity of macrophage inhibitory cytokine-1 (MIC-1) in a subject, said method comprising administering to said subject an effective amount of an agent which modulates the activity of a MIC-1 receptor complex comprising an RII receptor for transforming growth factor-β (TGF-β), optionally in admixture with a pharmacologically-acceptable carrier and/or excipient.

The invention also provides a method for screening candidate agents or libraries of agents for agents which modulate the activity of MIC-1.

Thus, in a second aspect, the present invention provides a method for screening an agent for capability to modulate the activity of macrophage inhibitory cytokine (MIC-1), said method comprising the steps of:
(i) contacting said agent with a MIC-1 receptor complex comprising the RII receptor for transforming growth factor-β (TGF-β), or a monomer thereof, and
(ii) detecting any change in the activity of said MIC-1 receptor complex or monomer thereof.

In a third aspect, the present invention provides a novel agent identified by the method for screening of the second aspect.

### BRIEF DESCRIPTION OF THE FIGURES

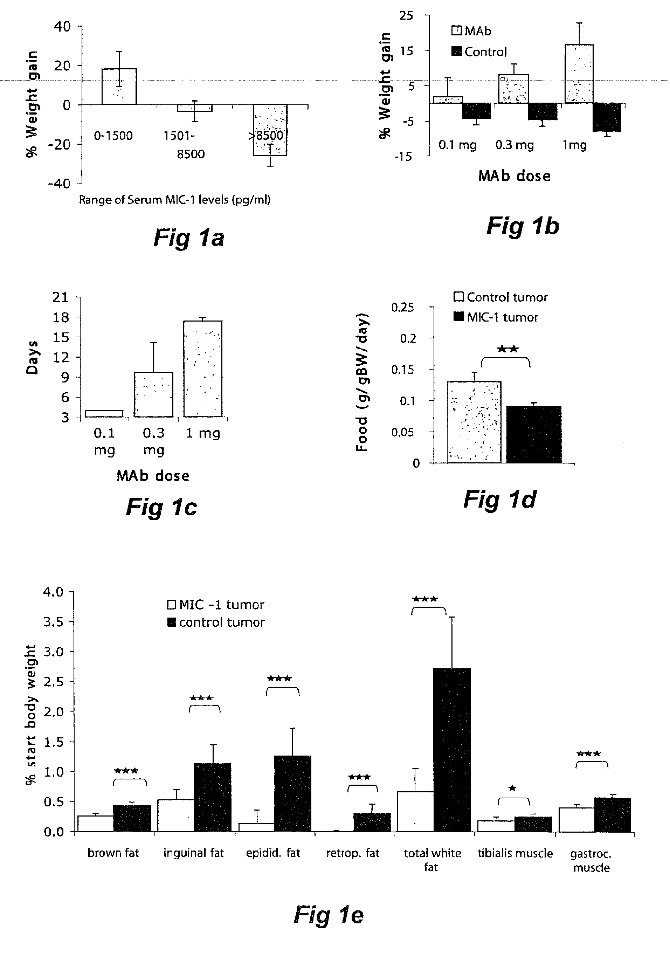

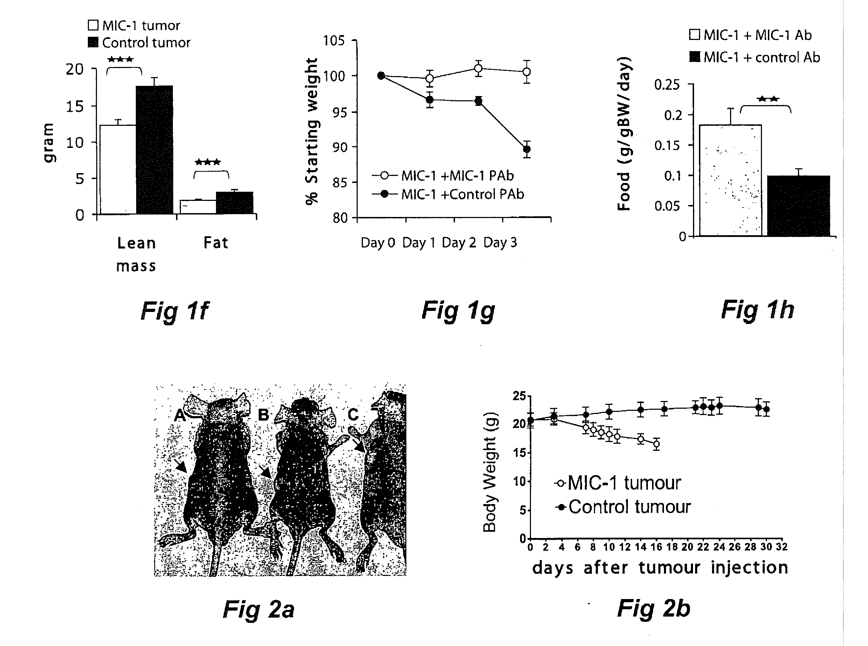

**Figure 1** shows: a. The effect on weight and tumour size in 45 nude mice injected subcutaneously in the flank with the DU145 human prostate cancer cell line, that had been permanently transfected with either a hMIC-1 expressing plasmid construct, or empty plasmid vector. The weight and tumour size of the mice was monitored for 6 weeks. At sacrifice, serum hMIC-1 levels were determined by ELISA and the mean and standard deviation of the degree of weight loss were plotted against serum level ranges of hMIC-1; b. The effect on mean weight change in nude mice xenografted with DU145 cells permanently transfected with a construct leading to high levels of MIC-1 in serum, of a single dose of MIC-1-MAb. After 11 days, when mice bearing the MIC-1 expressing tumour had lost a substantial amount of weight, three mice per dose were injected i.p. (i.p.), with a single dose of MIC-1-MAb and 5 mice were either injected control buffer (vehicle) or left untreated. Their weight was monitored daily for 20 days. Mean and standard deviation of their peak weight gain is shown plotted in comparison to the mean weight change in control mice over this same period; c. Shows the mean and standard deviation of the duration of the response of the mice as per "1b", defined as the time to return to their weight on the day of antibody injection, plotted against the dose of administered MIC-1-Mab; d. Shows weight change in 20 nude mice xenografted with DU145 cells permanently transfected with a construct leading to high levels of MIC-1 in serum and twenty with DU145 cells transfected with a control empty vector. On day 8 after injection, when the average weight loss in the MIC-1 mice was 7%, food intake was measured for 3 consecutive 24 hour time periods. Food was placed into the hopper and was weighed at time point 0. After 24hrs, food consumed was determined by subtracting refusal and spillage from food put into the hopper. The results are expressed as the mean and standard deviation of weight (grams) of food consumed per gram of mouse body weight per day. *=p<0.05, **=p<0.01, ***=p<0.001; e. 20 nude mice were engrafted with DU145 cells permanently transfected with a construct leading to high levels of MIC-1, and 20 with control empty vector construct. When the mice bearing MIC-1 tumours had lost approximately 18 % body weight, the mice were sacrificed. Interscapular brown adipose tissue, inguinal, epididymal, and retroperitoneal white adipose tissue, and also tibialis and gastrocnemius muscles were dissected, removed and weighed. Total white fat represents the summed weight of inguinal, epididymal, and retroperitoneal fat depots. The results are expressed as the mean and standard deviation of the specimen weight expressed as a proportion of the weight of the mouse at the day of tumour injection. *=p<0.05, **=p<0.01, ***=p<0.001; f. Prior to dissection, mice in "1.e" underwent DXA scanning to determine total lean and fat mass. The results shown are expressed as mean and standard deviation of total mass in grams. ***=p<0.001; g. 12 BALB/c mice were treated twice daily subcutaneously with 10 ug of highly purified recombinant hMIC-1. At the start of the experiment, half the mice were given a single i.p. dose of 10mg of MIC-1-PAb or CON-PAb. Mouse weight was monitored daily, expressed as a percentage of their starting weight and the results plotted as mean and standard deviation; and h. Shows daily food intake monitored for 3 consecutive 24 hour time periods on the hMIC-1 treated mice described in "1g". Food placed into the hopper and was weighed at time point 0. After 24hrs, food consumed was determined by subtracting refusal and spillage from food put into the hopper. The results are expressed as the average and standard deviation of weight (grams) of food consumed per gram of mouse body weight per day. *=p<0.05, **=p<0.01, ***=p<0.001.
**Figure 2** shows: a. A representative photograph of (A) nude mouse xenografted with DU145 cells transfected with empty vector (B) 14 days after xenografting of MIC-1 overexpressing DU145 cells (C) mouse treated as per "B" but given 9 days previously, 1mg i.p. of MIC-1-Mab. The arrow designates the site of tumour growth; b. Mean and standard deviation body weight of 40 nude mice xenografted with DU145 cells transfected with either a hMIC-1 expressing plasmid construct, or empty plasmid vector with MIC-1 and control tumour.

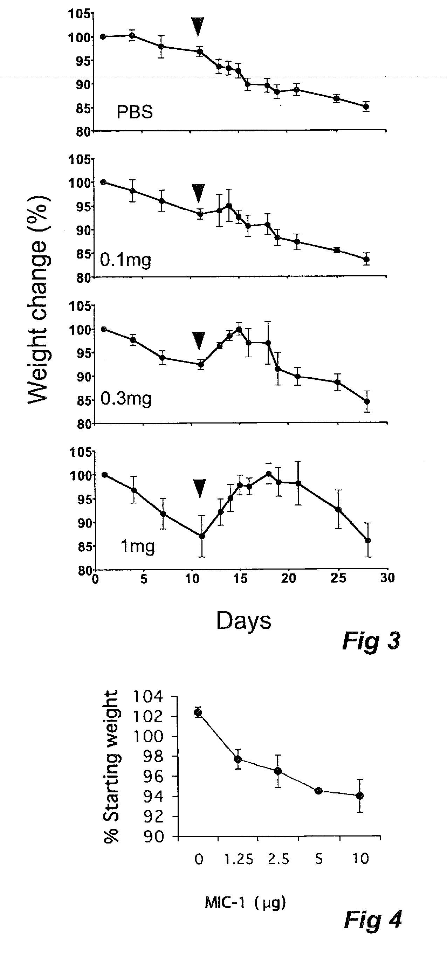

**Figure 3** provides results obtained from nude mice xenografted with DU145 cells permanently transfected with a construct leading to high levels of MIC-1 serum. After 11 days, when mice bearing the MIC-1 expressing tumour had lost a substantial amount of weight, three mice per dose were injected intraperitoneally, with 3 different doses of MIC-1-Mab or control buffer (PBS). Their weight was monitored daily for 20 days. For each group the mean and standard deviation of their relative weight change is plotted of the time over the course of the experiment. The time of injection ofMIC-1-Mab is marked with an arrowhead.
**Figure 4** shows the effect of MIC-1 dosage on weight loss. 18 BALB/c mice aged 9 weeks were injected subcutaneously twice daily with vehicle control or the indicated quantity of recombinant MIC-1. Their weight was monitored daily for 3 days and the weight change over this period calculated. The results are normalised to the body weight at the first day of injection of vehicle control and plotted as mean and standard error.

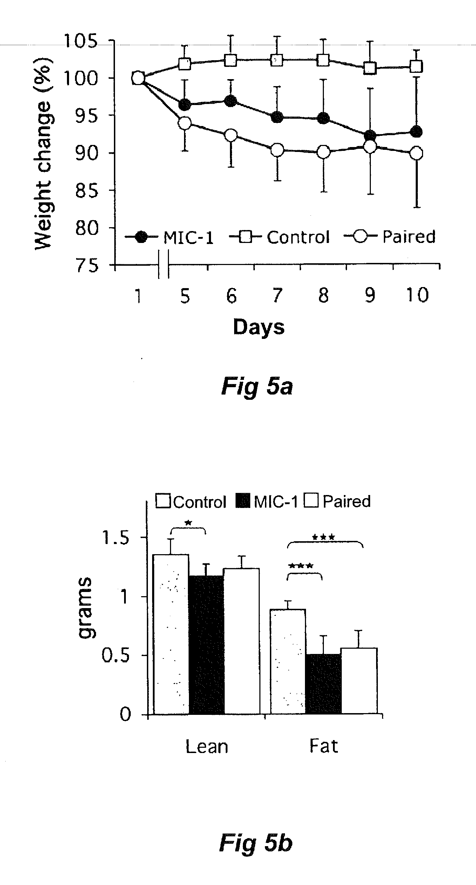

**Figure 5** shows: a. Change of weight of MIC-1 or vehicle (control) treated mice fed *ad libitum,* and pair fed vehicle treated animals (6 per group). The mice were injected subcutaneously twice a day with 10 µg of MIC-1 (or vehicle) for 10 days. The results are normalised to the body weight at the first day of MIC-1 injection and plotted as mean and standard deviation. The body weights of MIC-1 treated (p<0.05) and pair fed mice (p<0.001) are significantly reduced compared to vehicle treated controls. The difference between MIC-1 treated and pair fed mice was not significant (repeated measures two way ANOVA). b. After 10 days, the mice in 5a underwent DXA scanning to determine total body fat mass and lean mass of both hind legs. The results are expressed as the average (grams) and standard deviation. *=p<0.05, **=p<0.01, ***=p<0.001.

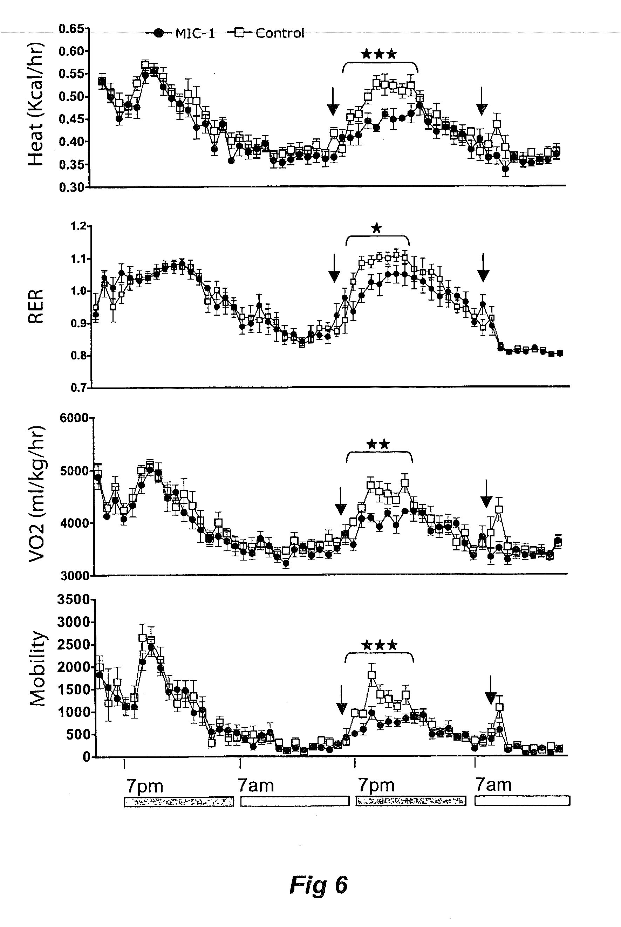

**Figure 6** shows the effect of MIC-1 on energy expenditure of mice. 15 BALB/c mice were housed individually in an open circuit calorimeter and acclimatised to the cages for 24 h. At the onset of the night cycle, 8 mice were injected (arrow) subcutaneously with 10 µg of MIC-1 and 7 mice with control buffer. A second injection (arrow) was given early in the next cycle, after which food was immediately withdrawn, in order to monitor MIC-1 induced metabolic changes in fasting state. Energy expenditure (HEAT), respiratory exchange ratio (RER), oxygen consumption and mobility (x and y total counts) were measured every cycle of 27 minutes. The 12 hour dark and light cycles are marked as horizontal bars. The data are average of 2 cycles and are plotted as mean and standard error of the mean. The data was compared by two-way repeated measures ANOVA. *=p<0.05, **=p<0.01, ***=p<0.001.

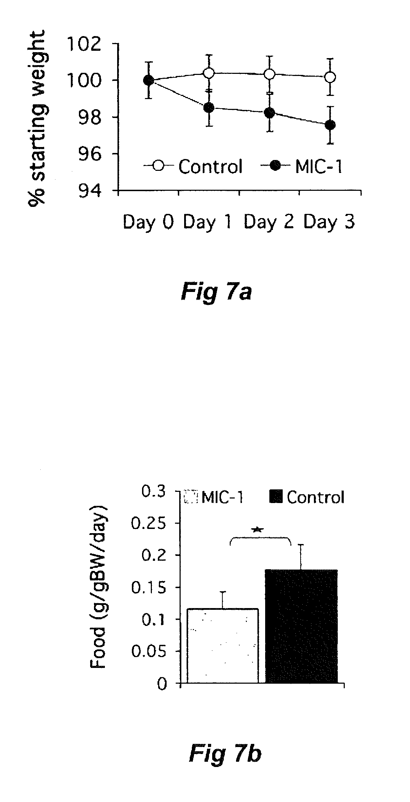

**Figure 7** shows the effect of MIC-1 on weight change and food intake of *ob*/*ob* mice. Leptin deficient *ob*/*ob* mice were injected subcutaneously twice daily with either vehicle control (5 mice) or 10 µg/20 g body weight of recombinant MIC-1 (5 mice). a. Body weight was measured daily and the results are normalised to the body weight at the first day of MIC-1 injection and plotted as mean and standard deviation. b. Food intake was measured on the last day and normalised for starting weight at the start of the experiment. The results were analysed using a 2 tailed t-test. *=p<0.05.

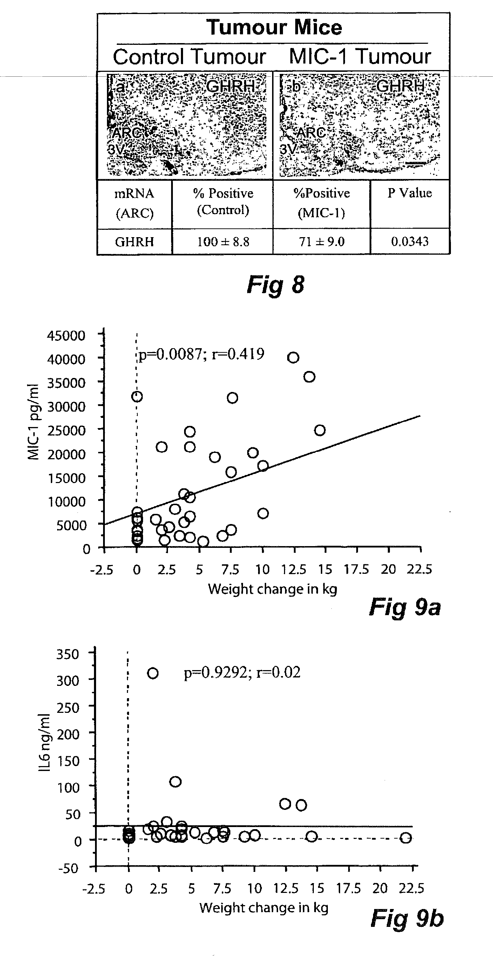

**Figure 8** provides images of emulsion autoradiographs of *in situ* hybridisation for GHRH mRNA expression in the hypothalamic arcuate nucleus of (a) a control tumour mouse and (b) a MIC-1 tumour mouse. Arc: arcuate hypothalamic nuclei; 3V the third ventricle. Scale bar = 40 µm.
**Figure 9** shows the correlation between serum levels of MIC-1 (a) or IL-6 (b) weight change in patients with advanced prostate cancer.

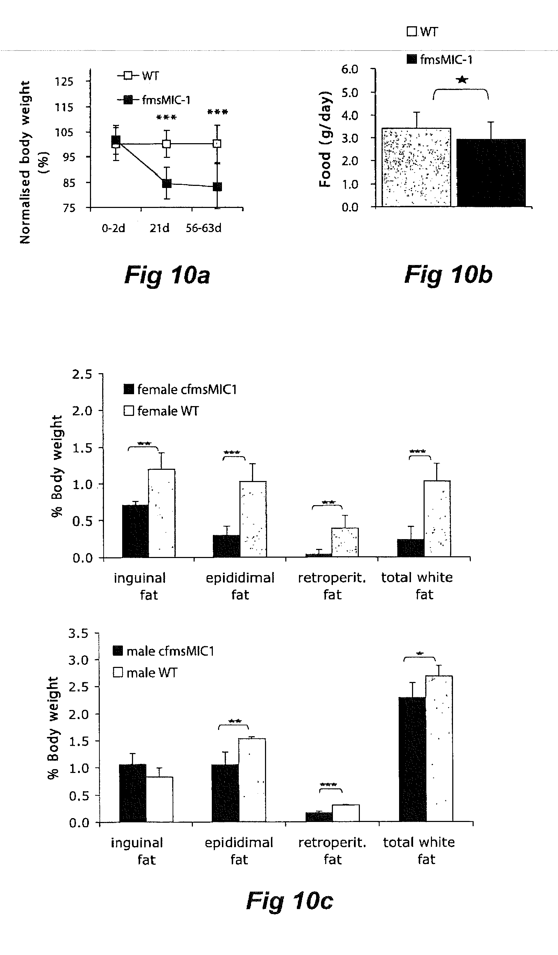

**Figure 10** shows: a. The weight of fmsMIC-1 transgenic mice and their WT littermates was measured ages after birth (0-48h, N=76), at 3 weeks (N=36) and at 8-9 weeks (N=40) of age. The weight has been normalised for each time point and sex. Results presented as the mean and standard deviation. *=p<0.05, **=p<0.01, ***=p<0.001. b. Daily food intake was monitored for 3 consecutive 24 hour time periods on 8 weeks old fmsMIC-1 and WT syngeneic control mice, as described in Figure 4a. The results are presented as the mean and standard deviation. *=p<0.05, **=p<0.01, ***=p<0.001; c. 5 male and 5 female fmsMIC-1 transgenic and 4 male and 5 female syngeneic control mice were sacrificed at 52-54 days of age. Fat depots and tibialis and gastrocnemius muscles were dissected, removed and weighed as per Figure 1e. The results are expressed separately for males and females, as the mean and standard deviation of the specimen weight expressed as a proportion of the weight of the mouse. *=p<0.05, **=p<0.01, ***=p<0.001.

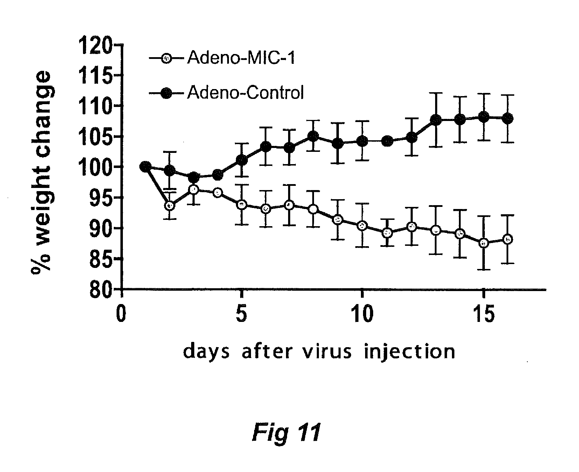

**Figure 11** provides results showing that direct AAV mediated overexpression of MIC-1 in the hypothalamus causes weight loss. Mice were given unilateral hypothalamic injection of AAV expressing MIC-1 or control AAV. Weight was monitored in 12 mice, half of whom received a unilateral hypothalamic injection of AAV overexpressing MIC-1 and the other half control AAV vector. Mice were weighed daily and the results plotted as mean and standard error of the mean.

### DETAILED DESCRIPTION OF THE INVENTION

It has previously been found that many cancers, especially of epithelial origin, overexpress MIC-1 leading to elevated circulating levels (ie serum levels) of MIC-1. These levels have been found to be in proportion to the stage and extent of the cancer disease and, in the case of late stage cancer, are particularly associated with marked weight loss. Similarly, it has now been found that with chronic renal failure, a condition which is commonly accompanied by weight loss and anorexia/cachexia, patients show markedly elevated serum levels of MIC-1 correlating with reduced body mass index (BMI). By reducing MIC-1 levels or the activity of MIC-1, it is expected that weight loss associated with cancer, chronic renal failure or other causes mediated by elevated MIC-1 serum levels, may be reversed or reduced. Indeed, in the above-mentioned International Patent Application No PCT/AU2005/000525 (WO 2005/099746), the present applicant demonstrated that anti-MIC-1 antibodies, which inhibit MIC-1 activity, are in fact capable of causing an increase in body weight in mice with M1C-1 overexpressing tumours. Achieving a reversal or reduction in weight loss is an important clinical outcome since this is expected to lead to improvements in patient well-being and esteem and, in turn, the capacity of patients to be successfully treated for the cancer, chronic renal failure or other causative disease or condition, as the case may be.

The present applicant has now undertaken an investigation of the mechanism of MIC-1 action which has led to an increased understanding of the pathway(s) and potential central regulators which effect modulation of appetite by MIC-1. More particularly, the MIC-1 receptor complex has now been characterised as comprising the TGF-β RII receptor. As a consequence, the present applicant has identified novel types of agents for modulating appetite and/or body weight, wherein the agents act by modulating the activity of the MIC-1 receptor complex. Moreover, the present applicant has identified methods of screening for novel types of agents which interact and modulate the activity of the MIC-1 receptor complex.

Thus, in a first aspect, the present invention provides method of modulating the activity of macrophage inhibitory cytokine-1 (MIC-1) in a subject, said method comprising administering to said subject an effective amount of an agent which interacts and modulates the activity of a MIC-1 receptor complex comprising an RII receptor for transforming growth factor-β (TGF-β), optionally in admixture with a pharmacologically-acceptable carrier and/or excipient.

Preferably, the agent interacts and modulates the activity of MIC-1 receptor complex, however the agent may otherwise act by modulating the amount of MIC-1 receptor complex.

By modulating the activity of MIC-1, the agent is capable of modulating appetite and/or body weight of a subject.

More particularly, by inhibiting (ie antagonising) the activity of MIC-1 (eg by binding to and blocking the MIC-1 receptor complex), the agent is capable of increasing appetite and/or body weight of a subject. In which case, the method is suitable for treating anorexia/cachexia associated with MIC-1 overexpression due to cancer, chronic renal failure or other causative disease, condition or treatment.

On the other hand, by enhancing (ie agonising) the activity of MIC-1 (eg by mimicking the activity of MIC-1 by binding to and stimulating the activity of the MIC-1 receptor complex), the agent is capable of decreasing appetite and/or body weight of a subject. In which case, the method may be suitable for treating, for example, obesity.

The receptors of the superfamily of receptors for TGF-β superfamily proteins (including MIC-1) are tetrameric complexes comprising two RI receptor monomers and two RII receptor monomers. The MIC-1 receptor is a tetrameric complex comprising two RI receptors and two TGF-β RII receptors. Numerous splice and other variants of TGF-β RII receptors exist (see, for example, the variants described by Konrad, L *et al*.³⁸) and it is, therefore, to be understood that the term "TGF-β RII receptor(s)", as used herein, encompasses such variants.

The agent utilised in the method of the first aspect may interact with one or both of the TGF-β RII receptor monomers and/or one or both of the RI receptor monomers. However, preferably, the agent interacts with one or both of the TGF-β RII receptor monomers. Preferably, the TGF-β RII receptor monomers are hypothalamic TGF-β RII receptor monomers.

Preferably, the method involves inhibiting the activity of MIC-1 and is conducted in order to increase appetite and/or body weight of the subject (who may be suffering from anorexia/cachexia associated with MIC-1 overexpression due to cancer (particularly an epithelial cancer such as, prostate, colon, pancreas and breast cancer), chronic renal failure or other causative disease (eg an inflammatory disease such as rheumatoid arthritis), condition (eg injury, inflammation or stress) or treatment (eg radiotherapy or chemotherapy)). For inhibiting the activity of MIC-1, the agent is preferably selected from agents which bind to and block the TGF-β RII receptor(s), preferably hypothalamic TGF-β RII receptor(s), although agents which bind to and block the RI receptor(s) or otherwise inhibit the interaction between the monomers of the tetrameric MIC-1 receptor complex, are also expected to be suitable.

Examples of suitable agents which bind to and block the TGF-β RII receptor include inhibitory peptide fragments and mimetics of MIC-1. However, preferably, such an agent is selected from the group consisting of anti-TGF-β RII antibodies (eg anti-TGF-β RII antibody AF532 available from R&D Systems, Inc., Minneapolis, MI, United States of America) and antibody fragments (eg Fab fragments and recombinant scFV fragments). More preferably, the agent is a humanised anti-TGF-β RII monoclonal antibody. Humanised anti-TGF-β RII monoclonal antibodies may be produced in accordance with the methods described in United States Patent No 5,225,539 (the entire disclosure of which is to be regarded as incorporated herein by reference).

Examples of suitable agents which bind to and block RI receptors include well known inhibitors such as ALK5 inhibitors including SB-431542 (4-(5-benzo(1,3)dioxol-5-yl-4-pyridin-2-yl-1*H-*imidazol-2-yl)benzamide) (Sigma Aldrich, St Louis, MO, United States of America), SB-505124 (2-(5-benzo[l,3]dioxol-5-yl-2-tert-butyl-3H-imidazol-4-yl)-6-methylpyridine hydrochloride)³⁵, SD-208³⁶ (Scios, Inc., Freemont, CA, United States of America), and GW6604 (2-phenyl-4-(3-pyridin-2-yl-1H-pyrazol-4-yl)pyridine)³⁷ and inhibitory peptide fragments and mimetics of MIC-1. However, preferably, such an agent is selected from the group consisting of anti-RI antibodies and antibody fragments (eg hAP-0016 antibody against ALK5 extracellular domain, available from Angio-Proteomie, Boston, MA, United States of America). More preferably, the agent is a humanised anti-RI monoclonal antibody.

Subjects suitable for treatment with the method of the first aspect, when conducted so as to inhibit the activity of MIC-1, may be restricted to those showing MIC-1 overexpression or, at least, a serum level of MIC-1 consistently at the high end of the normal human serum level of 200-1200 pg/ml. Such subjects can be selected by detection of a high serum MIC-1 level (eg from a whole blood or serum sample), using an assay for MIC-1 (eg a MIC-I ELISA³³). A "high" serum MIC-1 level includes > 850 pg/ml, more preferably > 1000 pg/ml and, most preferably, > 1200 pg/ml.

Alternatively, the method involves enhancing the activity of MIC-1 and is conducted in order to decrease appetite and/or body weight of the subject (who may be suffering from obesity or who might otherwise desire weight loss for reasons of well being or vanity). For enhancing the activity of MIC-1, the agent is preferably selected from agents which mimick the activity of MIC-1 by binding to and stimulating the activity of TGF-β RII receptor(s), although agents which bind to and stimulate the activity of RI receptor(s) are also expected to be suitable. Examples of suitable agents which bind to and stimulate the TGF-β RII receptor include stimulatory peptide fragments and mimetics of MIC-1, and examples of suitable agents which bind to and stimulate RI receptors include stimulatory peptide fragments and mimetics of MIC-1.

Further, the method of the first aspect may involve modulating the activity of MIC-1 by modulating the amount of MIC-1 receptor complex (ie the amount of endogenous MIC-1 receptor complex in the subject). For modulating the amount of MIC-1 receptor complex, the agent is preferably selected from agents which decrease the amount of MIC-1 receptor complex in the subject, and may be selected from catalytic and inhibitory oligonucleotide molecules targeted against the gene(s) encoding the MIC-1 receptor complex (eg ribozymes, DNAzymes, antisense RNA, and small inhibitory RNA (siRNA)), and inhibitors of MIC-1 receptor complex transcription or translation. Preferably, such an agent decreases the amount of endogenous TGF-β RII receptors, preferably endogenous hypothalamic TGF-β RII receptors, although an agent that decreases the amount of endogenous RI receptors may also be suitable. Such agents are expected to be suitable for treating anorexia/cachexia associated with MIC-1 overexpression due to cancer, chronic renal failure or other causative disease, condition or treatment.

Agents for use in the method of the first aspect may be formulated into any suitable pharmaceutical/veterinary composition or dosage form (eg compositions for oral, buccal, nasal, intramuscular and intravenous administration). Typically, such a composition will be administered to the subject in an amount which is effective to modulate appetite and/or body weight, and may therefore provide between about 0.01 and about 100 µg/kg body weight per day of the agent, and more preferably providing from 0.05 and 25 µg/kg body weight per day of the agent. A suitable composition may be intended for single daily administration, multiple daily administration, or controlled or sustained release, as needed to achieve the most effective results.

Further, the invention also provides a method for screening candidate agents or libraries of agents for agents which modulate the activity of MIC-1.

Thus, in a second aspect, the present invention provides a method for screening an agent for capability to modulate the activity of macrophage inhibitory cytokine (MIC-1), said method comprising the steps of:
(i) contacting said agent with a MIC-1 receptor complex comprising the RII receptor for transforming growth factor-β (TGF-β), or a monomer thereof, and
(ii) detecting any change in the activity of said MIC-1 receptor complex or monomer thereof.

Preferably, said method is conducted *in vitro* using, for examples, cultured cells (eg CHO cells) expressing the MIC-1 receptor complex or a monomer thereof. Changes in the activity of the MIC-1 receptor complex or monomer thereof may be detected by detecting the activation (eg by phosphorylation), or lack of activation, of one or more signalling molecules such as erk 1/2, P-stat3, and smad signalling pathways. Alternatively, changes in the activity of the MIC-1 receptor complex or monomer thereof may be detected by detecting the activation, or lack of activation, of one or more signalling molecules of the akt signalling pathway which has been previously reported as being associated with MIC-1 activity³⁴.

Where step (i) of the method is conducted using a monomer of the MIC-1 receptor complex, preferably the monomer is the TGF-β RII receptor, more preferably, the hypothalamic TGF-β RII receptor.

A detected decrease in the activity of the MIC-1 receptor complex or monomer thereof may indicate that the agent is likely to increase appetite and/or body weight of a subject. On the other hand, a detected increase in the activity of the MIC-1 receptor complex or monomer thereof may indicate that the agent is likely to decrease appetite and/or body weight of a subject.

In a third aspect, the present invention provides a novel agent identified by the method of the second aspect.

The invention is hereinafter further described by way of reference to the following non-limiting examples and accompanying figures.

### EXAMPLES

### Example 1

### Materials and Methods

### Tumour xenograft model

Eight-week-old BALB/c nude mice were injected subcutaneously on their right flank, with 5-7 x 10⁶ transfected DU145 cells in 200ml of PBS. The DU145 cells had been transfected with either the pIRES2-EGFP plasmid (Clontech, Mountain View, CA, United States of America) containing mature MIC-1 (MIC-1), full length MIC-1, a proconvertase site deletion mutant of MIC-1, or empty vector¹¹. Once visible, the tumour size was measured and mice were weighed every 1-3 days for 6 weeks.

Lean and fat mass was determined by both dual X-ray absorptiometer (DXA) (Lunar Piximus II, GE Medical Systems, Madison, WI, United States of America) and by dissection of white and brown adipose tissue depots, tibialis and gastrocnemius muscles, then expressing their weight as a proportion of the body weight of the mouse on the day of the tumour injection

### Generation of MIC-1 overexpressing transgenic mice

Transgenic mice were constructed by conventional methods. Briefly, a plasmid construct containing a modified c-fms promoter sequence driving expression, in a macrophage specific manner, of the full-length coding sequence for murine MIC-1 was linearised and injected into C57BL6 oocytes. The resulting transgenic mice were then mated with WT C57BL6 mice and the resultant transgenic mouse lines, called fmsMIC-1, selected based upon an increase in MIC-1 expression in spleen (a macrophage rich tissue) as assessed by real time RT-PCR. MIC-1 was strongly expressed to the extent that it was readily detectible in mouse serum.

### MIC-1 reagents

All MIC-1 antibodies and recombinant protein were prepared and purified as previously described^{11,15,16}. Recombinant MIC-1 was expressed in and purified from conditioned medium of the yeast *Pichia pastoris* using a combination of hydrophobic interaction chromatography, ion exchange chromatography then reverse phase HPLC. MIC-1 PAb or control IgG was prepared by protein G affinity chromatography of serum from sheep that were either immunised with recombinant MIC-1 or unimmunised, respectively. For some experiments, affinity purified anti-MIC-1 antibody was prepared by purifying MIC-1 PAb on a sepharose column to which had been coupled purified recombinant MIC-1. Monoclonal antibody to MIC-1 was derived from hybridoma and purified by protein G affinity chromatography.

### Production of AAV vector engineered to express MIC-1

The cDNA for human MIC-1 was sub-cloned into an AAV expression plasmid under the control of a CBA (chicken β-action) promoter and containing WPRE (woodchuck hepatitis virus post-transcriptional-regulatory element), and bovine growth hormone plyadenylation signal flanked by AAV2 inverted terminal repeats (ITRs). AAV expression plasmid without transgene insert was packaged into AAV and used as control vector. Infectious AAV particles were then produced essentially as described³². In brief, HEK293 cells were co-transfected with AAV and helper plasmids using standard CaPO₄ transfection. Cells were harvested 60hr following transfection, and AAV vectors purified from the cell lysate by ultracentrifugation through an iodixanol density gradient, then concentrated and dialysed against PBS. Vectors were titred using real-time PCR (ABI Prism 7700) and all vector stocks diluted 1 x 10¹³ genomes/ml.

### Determination of metabolic rate, respiratory exchange ratio and physical activity

Metabolic rate was measured by indirect calorimetry in either 8-10 week old male BALB/c, fmsMIC-1 or C57B16 mice using an eight-chamber open-circuit calorimeter (Oxymax Series, Columbus Instruments, Columbus, OH, United States of America). Mice were housed individually in specially built Plexiglas cages (20.1cm x 10.1cm x 12.7cm). Temperature was maintained at 22°C, with an air-flow of 0.6 l/min. Food and water were either available *ad libitum* or in some experiments, mice were deprived of food for the 12h following injection of MIC-1. Mice were acclimatised to the cages for 24 h before beginning recordings. Mice were then injected subcutaneously with either 10 ug of MIC-1 or control buffer, then monitored for 12 h. Oxygen consumption (VO2 was measured every 27 minutes, and the respiratory exchange ratio (RER) was calculated as *V*CO2/*V*O2. Energy expenditure (kcal heat produced) was calculated as Calorific Value (CV) x VO2, where CV is 3.815 + 1.232 x RER. Statistical analysis of data for heat production was by repeated measures two-way ANOVA.

### Pair-feeding experiment

Experiments were performed using 3 groups of 8 week old, female, BALB/c mice. The mice were individually housed for one week before the start of the experiment. One group of mice were treated twice daily with subcutaneous injections of 10 µg of MIC-1 and fed *ad libitum.* A second group of control mice were injected with vehicle only and also fed *ad libitum.* The third and pair fed group, were injected with vehicle only and pair fed to the MIC-1 injected mice.

Pair feeding was done by measuring the food consumed by the MIC-1 treated group every 24 hours. The following day, the pair fed mice were given the same amount of food as was consumed by their MIC-1 treated pair on the previous day. To measure food intake the pelleted food was weighed and placed into the food hopper; the food remaining 24 hours later was weighed again and the difference corrected for spillage and faeces represented the daily food intake.

### Cancer cachexia patient cohort

Serum samples for estimation of MIC-1 levels were obtained from patients enrolled in a previously published study of cancer cachexia²³. Briefly, this represented a control group of 10 patients with no prostatic disease, a group of 19 patients newly diagnosed with organ-confined cancer of the prostate (CaP) and a group of 38 patients with advanced CaP. For further analysis, this group was divided into two subgroups: advanced prostate cancer without cachexia and with cachexia. Advanced prostate cancer patients were categorised based on clinical status as determined by an oncologist prior to the study entry. A patient was determined to be cachectic when he presented with loss of weight over a 3-6 month period and loss of appetite (anorexia). If both symptoms were absent, the patient was considered to be non-cachectic. If a single symptom was present, cachexia was only diagnosed when one of the symptoms of low energy level, fatigue, or low exercise tolerance, were present. The advanced PCa without cachexia subgroup consisted of 14 patients. Weight loss was seen in 3/14 patients with a mean ± SEM weight loss of 0.87 ± 0.51 kg (median: 0 kg; range: 0-6.2 kg). Despite the weight loss in some patients they were not considered to be cachectic according to their clinical status as determined by the attending oncologist. The median age was 72.5 years. The subgroup with cachexia consisted of 24 patients. Only one patient was placed in the cachectic group on the basis of the secondary criteria. All patients in this group had lost weight; mean ± SEM weight loss of 6.89 ± 1.00 kg (median: 4.7 kg; range: 1.5-21.9 kg). The median age was 68.5 years

### Mouse intrahypothalamic injection

All experiments were undertaken using the following procedure. Mice were anesthetised with 100 mg/kg keatmine and 20 mg/kg xylazine (Parke Davis-Pfizer, Sydney, Australia and Bayer AG, Leverkusen, Germany) and placed on a Kopf stereotaxic frame (David Kopf, CA, United States of America), with the head in the flat skull position. Mice were injected unilaterally into the hypothalamus with the relevant substance at a rate of 0.1 µl/min using a 10µl Hamilton syringe attached to Micro4 Micro Syringe Pump Controller (World Precision Instruments Inc., Sarasota, FL, United States of America).

For direct injection of cytokines, adult WT mice were injected with 1µl MIC-1, 1µl leptin or 1µl control solution using coordinates relative to Bregma of posterior 2.3 mm, lateral ± 0.3 mm, ventral 5.6 mm, which corresponded to the ARC²⁸. Thirty minutes after the start of injection, mice were sacrificed. The brains were fixed by perfusion and P-stat3 neurons were identified by immunohistochemistry as below.

### Administration of anti-TGF-β receptor antibodies

For experiments involving injection of anti-TGF-β receptor antibodies, 1.5µl of antibody (0.1mg/ml) was injected unilaterally into the posterior hypothalamic area of 3 adult mice at a rate of 0.1µl/min. The injected antibody was inhibitory goat antibody to TGF-β RII (R&D Systems, Inc.) or BMP RII (GeneTex, Inc., San Antonio, TX, United States of America) or leptin receptor (Upstate Biotech., Lake Placid, NY, United States of America). In this instance, the needle was left in place for 10 minutes after injection to allow diffusion. The injection coordinates were (from bregma): anterio-posterior, -2.3 mm; medio-lateral, -0.5 mm; dorso-ventral, -4.5 mm corresponding to the posterior hypothalamic area²⁹. Twenty-four hours after TGF-β RII antibody injection, animals were injected i.p. with 100µg of hMIC-1 at 10am to 12 pm. Exactly 30 minutes after injection, the mice were sacrificed and brain collected for detection of c-fos and TGF-β RII immunoreactive neurons in the posterior hypothalamic area as described below.

In an alternative experiment, six adult male mice were divided into three groups: 1.5µl anti-TGF-β RII antibody (0.1mg/ml) (R&D Systems, Inc.), 1.5µl anti-BMPRII antibody (0.1mg/ml)(GeneTex Inc., San Antonio, TX, United States of America) or 1.5µl anti-leptin antibody (0.1 mg/ml) (Upstate Biotech.) were injected unilaterally into the ARC. The needle was left in place for 10 minutes after injection for 10 minutes to allow diffusion. The injection coordinates were (from bregma): anterio-posterior, -1.94mm; medio-lateral, -0.4mm; dorso-ventral, -5.5mm corresponding to the arcuate hypothalamic nucleus²⁹. Twenty-four hours after injection, animals were injected i.p. with 100µg of hMIC-1 at 10am to 12 pm and 30 minutes after injection, the mice were sacrificed and brains were collected for detection of c-fos immunoreactivity in the arcuate hypothalamic area as described below.

### Immunohistochemistry and immuofluorescence of mouse brain

BALB/c mice were injected i.p. with either 10µg or 100µg of hMIC-1 or control buffer at a fixed time of the day (10am to 12pm). To ensure the least possible variation in c-fos immunoreactivity, all mice were sacrificed, exactly 60±1 minutes after i.p. injection of 10µg of hMIC-1. For P-stat3, mice were treated with 5 mg/kg (about 100µg) of leptin, 100µg of hMIC-1 or control buffer and mice were sacrificed, exactly 30 minutes after i.p. injection. Mice were deeply anaesthetised, and the brain were fixed by perfusion with 25 ml 0.9% saline in phosphate buffer, following by cold 4% paraformaldehyde made in 0.1 M phosphate buffer (PH 7.4). The brains were immediately removed and placed in 4% paraformaldehyde for 30 minutes and then in 30% sucrose solution in phosphate buffer overnight. The brains were sectioned into 30 µm coronal slices and washed in 1% H₂O₂ in 50% alcohol for 20 min to abolish endogenous peroxidase activity. The primary antibody, rabbit anti-mouse c-Fos (sc-52; Santa Cruz Biotechnology Inc., Santa Cruz, CA, United States of America), was diluted at 1:5000 in PBS containing 0.1% Triton x-100 and then incubated overnight. The rabbit-anti-mouse Phospho-Stat3 (Cell Signaling Technology Inc., Danvers, MA, United States of America), rabbit anti-phosphoERK1/2 antibody (Cell Signaling) and goat-anti-mouse TGF-β RII (R&D Systems, Inc.) were treated similarly except that they were diluted at 1:500, 1:2000 and 1:2000 respectively. After three washes in PBS-Triton for 10-min each, sections were incubated for 2 h with the biotinylated anti-rabbit (Sigma Aldrich) or anti-goat secondary antibody (Chemicon, Temecula, CA, United States of America) diluted 1:250 in PBS. Sections were washed in PBS for 30 min and then incubated with the Avidin-Biotin-Peroxidase (Vectastain, Burlingame, CA, United States of America) for 30 minutes at room temperature. For some experiments, where nickel enhanced staining was used to differentiate reactivity of two antibodies on the one section, sections were washed in PBS and then treated with 0.05% diaminobenzidine tetrahydrochloride (Sigma) and 0.007% hydrogen peroxide in the presence for 0.04% nickel ammonium sulphate. Sections were then washed in PBS and treated with diaminobenzidine from DAKO (Carpinteria, CA, United States of America) for 5 minutes. Slides were rinsed in water, dehydrated through to xylene before coverslipping. Nuclear Fos-positive neurons were counted with a grid reticule and 10x objective of a Zeiss Axioplan light microscope. In the regions including where differences were readily apparent, c-fos positive nuclei were counted. For each brain nucleus of interest, every third section was counted to quantify of c-fos positive neurons, according to the atlas of mouse brain in stereotaxic coordinates by Franklin and Paxinos²⁹. Comparisons were made between the two groups (5 mice per group) stained at same time and the average number of neuron counts in each nucleus was determined from both left and right sides. All groups were pooled for final analysis and the differences between groups were assessed by ANOVA followed by Fisher's post hoc tests.

For immunofluorescence, the primary antibody, rabbit-anti-mouse P-Stat3 (Cell Signaling) and sheep anti-mouse α-MSH (AB5087; Chemicon) were diluted at 1:5000 and 1:4000 in phosphate-buffered saline (PBS) containing 0.1% Triton x-100 and then incubated overnight. After three washings in PBS-Triton for 10-min each, sections were incubated in a goat anti-rabbit and a donkey anti-sheep secondary antibodies conjugated to Alexa fluorophores (Alexa Fluor ® 594 and Alexa Fluor ® 488; Molecular Probes, Eugene, OR, United States of America), diluted at 1:250 in PBS for 3 h. Sections were washed three times in PBS for 10 min each and fluorophore signals (red colour for c-Fos and green colour for α-MSH neurons) were checked under a Zeiss Axioplan light microscope (Oberkochen, Germany).

### In situ hybridisation

Mice were injected i.p. with either 10µg of hMIC-1 or control buffer at a fixed time of the day (10am to 12 pm). All mice were sacrificed, exactly 60 minutes after i.p. injection and brains removed immediately and frozen on dry ice. Coronal sections (20 µm) were cut on a cryostat and thaw-mounted on Superfrost® slides (Menzel-Glaser, Braunschweig, Germany). Matching sections from the same coronal brain level of MIC-1 injection and PBS injection of mice (n=5 mice/group) were assayed together using radiolabelled DNA oligonucleotides complementary to mouse *NPY* (5'-GAGGGTCAGTCCACACAGCCCCATTCGCTTGTTACCTAGCAT-3') (SEQ ID NO: 1); mouse *POMC (5'-*TGGCTGCTCTCCAGGCACCAGCTCCACACATCTATGGAGG-3') (SEQ ID NO: 2); mouse GHRH (5'-GCTTGTCCTCTGTCCACATGCTGTCTTCCTGGCG GCTGAGCCTGG-3') (SEQ ID NO: 3), as described previously²⁸.

For evaluation of mRNA levels in scattered neurons, images from dipped sections were digitised using a ProgRes 3008 camera (Zeiss, Jena, Germany) mounted on a Zeiss Axiophot microscope. Silver grain density over single neurons was evaluated using NIH-Image 1.61 software (written by Wayne Rasband and available from anonymous FTP at zippy.nimh.nih.gov). Background labelling was uniform and never exceeded 5 % of specific signal.

### Combined immunohistochemistry (in situ hybridisation)

C57B6 mice were treated with recombinant hMIC-1 as outlined above and identically processed for P-stat3 immunohistochemistry. Immunohistochemical labelling for P-stat3 was carried out as previously outlined with minor variation. Anti-P-stat3 antibody was diluted 1:2000 in PBS containing 0.3% Triton X-100, 0.1% BSA and heparin 5mg/ml. Sections were incubated for 48hrs at 4°C and specific antibody labelling detected using the same procedure as above. Sections were mounted and NPY *in-situ* hybridisation performed as outlined above.

### Statistical analysis.

Prostate cancer patient data were analysed using Mann-Whitney-U testing in addition to linear, univariate logistic and multivariate logistic regression. Comparison of serum MIC-1 with IL-6 as stand-alone tests for the prediction of cancer associated weight loss was undertaken using McNemar's test (GraphPad, San Diego, CA, United States of America). Comparison of metabolic data was done by two way repeated measures ANOVA using PRISM 4. Unless otherwise specified all other statistical evaluation of differences was undertaken using an unpaired two-tailed T-test. All error bars on graphs signify standard deviations, unless otherwise specified. Unless otherwise stated, all analysis was performed using STATVIEW VERSION 4.5 (Abacus Concepts, Berkeley, CA, United States of America).

### Results

### MIC-1 induces rapid weight loss that is reversible by antibodies to MIC-1

In order to understand the effects of both local and systemic overexpression of MIC-1, the human prostate cancer cell line DU145, which constitutively makes no MIC-1, was permanently transfected with human MIC-1 (hMIC-1) expressing plasmid constructs. These constructs either retained or were missing the propeptide domain, which mediates matrix binding of MIC-1¹¹. Transfected DU145 cell lines were injected subcutaneously into the flanks of nude mice to establish xenografted tumours". The mice were monitored for 6 weeks, after which serum hMIC-1 levels were determined using a highly specific ELlSA^{11,15,16}. The mice with elevated tumour derived hMIC-1, progressively lost weight in proportion to end point serum MIC-1 concentrations (Figures 1a, 2a and 2b). Mice with low serum levels of tumour derived hMIC-1 (0-1500 pg/ml), similar to those found in normal humans, gained about 19% body weight, consistent with the young age of these mice (Figure 1a). Mice with moderately elevated levels of MIC-1 (1501-8500 pg/ml) lost about 3% of their body weight and those with markedly elevated MIC-1 levels (>8500 pg/ml) lost about 28% body weight over the course of the experiment (Figure 1a). As mice with control tumours continued to gain weight over the course of the experiment, the net effect of MIC-1 with respect to the control group was a 22% and 47% reduction in weight in mice with moderate and high serum levels of hMIC-1, respectively.

To directly demonstrate the specificity of the effects of MIC-1 on weight loss, its actions were reversed by injecting tumour-bearing mice with polyclonal sheep anti-MIC-1 IgG (MIC-1-PAb). This reversal of the weight-reducing effect of MIC-1 was not seen when using control IgG (CON-IgG) (data not shown). Additionally, a single injection of a monoclonal antibody specific for hMIC-1 (MIC-1-MAb), but not vehicle control, rapidly reversed this MIC-1 related weight loss (Figures 1b, 1c, 2a and 3) any effect on tumour size (data not shown). The magnitude and duration of reversal of MIC-1 induced weight loss was proportional to the amount of administered MIC-1-MAb. Injection of 1mg of MIC-1-MAb lead to a peak weight gain of 14%, which resulted in complete reversal of tumour induced weight loss. This compared with marked continued weight loss in the MIC-1 tumour bearing mice administered the vehicle control (Figure 3). The duration of the effect of this MIC-1-MAb was 17 days (Figure 1c). Again, antibody treatment had no effect on tumour size (data not shown). These data indicates that weight loss induced by MIC-1-expressing tumours is specifically mediated by MIC-1.

To further demonstrate that MIC-1 was responsible for this effect, increasing doses of purified recombinant hMIC-1 were administered subcutaneously twice daily to normal BALB/c mice for 3 days and their weight change over this period measured. This demonstrated that MIC-1 induced weight loss in a dose dependent manner (Figure 4), with a 10 µg dose of purified recombinant hMIC-1 resulting in a marked and immediate weight loss that could be completely reversed by giving mice MIC-1-PAb, but not CON-PAb (Figure 1g).

### Characterisation of MIC-1 induced weight loss in mice

To more specifically characterise the nature of the weight loss induced by MIC-1 expressing tumours, changes were determined in the fat and lean tissue mass of various areas by dissection of white and brown adipose tissue depots, tibialis and gastrocnemius muscles, then expressing their weight as a proportion of the body weight of the mouse on the day of the tumour injection (Figure 1e). Mice bearing tumours overexpressing MIC-1 had no remaining retroperitoneal fat and a 54% and 89% reduction in inguinal and epididymal fat depots, respectively. There was a smaller but significant reduction in the weight of the interscapular brown adipose tissue depot (40%) as well as tibialis and gastrocnemius muscles (25% and 29%, respectively) of MIC-1 tumour bearing mice compared to mice bearing control tumours. Dual Energy X-ray Absorptiometry (DXA) scanning data confirmed that MIC-1 tumour bearing mice lost an average of 5.3g of lean mass and 1.1g of fat mass compared to controls (Figure 1f).

To determine whether the weight loss in mice bearing MIC-1 overexpressing tumours was due to decreased feeding, food ingestion was measured for 3 successive days. Mice with DU145 cell tumours associated with high serum levels of MIC-1 were compared with mice bearing control DU145 cell tumours transfected with empty vector. Mice bearing tumours overexpressing MIC-1 ate, on average, 32% less per day than their control tumour bearing counterparts (Figure 1d). Food consumption was also measured in BALB/c mice injected with recombinant MIC-1, which was administered with either MIC-1-PAb or CON-PAb. Similar to the data from the tumour bearing mice, administration of recombinant MIC-1 induced a marked decrease in food intake (Figure 1h). Importantly, this hypophagia was reversed by MIC-1-Pab treatment leading to weight gain (Figure 1h), establishing decreased food intake as a major component of the weight loss mediated by MIC-1.

To discover whether MIC-1-induced loss of fat mass, lean mass and body weight are due entirely to hypophagia or whether metabolic factors also play a role, vehicle-injected mice were pair-fed to the amount of food consumed by mice receiving twice-daily injections of MIC-1. Pair-fed vehicle-injected mice lost at least as much weight as MIC-1 injected animals and showed no significant difference from MIC-1 injected mice with respect to loss of lean or fat mass (Figure 5a and 5b). The slightly greater weight loss observed in pair-fed animals is most likely due to the induced stress response and increases in physical activity (eg food-seeking behaviour) caused by the food restriction¹⁷ whereas no such increase in physical activity would have occurred after MIC-1 injection. Taken together, these data establish hypophagia as the means by which MIC-1 reduces body weight and the associated loss of fat and lean mass.

To completely exclude increased metabolic rate as a contributor to weight loss and changes in body composition in MIC-1 treated mice, energy expenditure was investigated by indirect calorimetry in normal mice treated with a single injections of control buffer or recombinant MIC-1. Compared to controls, mice injected with 10µg of MIC-1 showed a small, but significant reduction in energy expenditure (Figure 6). When mice were fasted over the 12 hours following MIC-1 injection, this small relative decrease in energy expenditure disappeared (Figure 6). Thus, increased energy expenditure cannot account for the weight loss and altered body composition in MIC-1 treated mice, further supporting decreased energy intake as the sole cause.

In order to test if MIC-1 also has an effect in obese animals, genetically obese leptin-deficient *ob*/*ob* mice were injected with 10µg of MIC-1 per 20g of body weight, twice daily for 3 days. Similar to effects of MIC-1 in lean mice, *ob*/*ob* animals showed significant reductions in body weight (Figure 7a). This weight reduction was consequent to a significant decrease in food intake in the MIC-1 injected *ob*/*ob* mice compared to control injected mice (Figure 7b). These data show that MIC-1 powerfully induces hypophagia and weight loss, even in a massively obese animal model.

Evaluation of serum levels of hormones and metabolites showed that MIC-1 tumour bearing mice had substantial reductions in serum levels of free fatty acids, triglycerides, glucose, glucagon, leptin, and IGF-1 (data not shown), consistent with their loss of lean and fat mass. MIC-1 tumour bearing mice also showed tendencies to increases in serum corticosterone levels. The changes in circulating IGF-1 levels suggest an inhibition of the hypothalamo-pituitary somatotropic axis and consequently growth hormone releasing hormone (GHRH) mRNA expression was measured in the hypothalamus. As shown in Figure 8, the level of GHRH expression in the brain of MIC-1 tumour-bearing mice is strongly reduced, demonstrating centrally-mediated down-regulation of the somatotropic axis in these animals. While reduced serum IGF-1 levels may contribute to some of the effects of MIC-1, notably loss of lean tissues¹⁸, this neuroendocrine change cannot account for the fat loss seen in MIC-1 treated animals. Indeed, congenic mice with low serum IGF-1 levels show significantly increased body fat¹⁹.

Inhibition of the somatotropic axis is likely to be a protective adaptation to the anorexia and weight loss caused by MIC-1. In keeping with this, restricted energy intake inhibits the hypothalamo-pituitary-somatrotropic axis and/or reduces serum IGF-1 levels in rodents and in humans^{20,21,22}. Therefore, the effects of MIC-1 treatment on the somatotropic axis are likely a secondary consequence of the primary effects of MIC-1 (anorexia and weight loss) rather than primary mediators of the effects of MIC-1.

MIC-1 tumour-bearing mice also exhibited a significant reduction in serum leptin levels, despite concurrent hypophagia. This suggests that MIC-1 overrides the orexegenic effects of reduced serum leptin levels and is consistent with the observation that leptin-deficient *ob*/*ob* mice, like lean animals, also demonstrate hypophagia and weight loss in response to MIC-1.

### Serum MIC-1 levels directly correlate with weight loss in advanced prostate cancer patients

To determine the relevance of MIC-1 to anorexia/cachexia in humans, serum MIC-1 levels were measured in patients recruited into a well-characterised cohort of patients with advanced prostate cancer (PCa), in which serum IL-6 levels had been associated with cachexia²³. Serum MIC-1 levels were significantly elevated in patients with advanced cancer with cachexia compared to those with advanced cancer without cachexia (12416±10235pg/ml vs 3265±6370 pg/ml (mean±SD); p<0.0001; Mann-Whitney-U test). Similarly, serum levels of IL-6 were elevated in cachexia patients (33.8 ± 64.2 pg/ml *vs* 7.8 ±3.4 pg/ml, p<0.002; Mann-Whitney-U test). Serum MIC-1 was weakly but significantly and positively correlated with serum IL-6 levels (r=0.2949, p<0.04; linear regression). Additionally, in single and multivariate logistic regression, both serum MIC-1 and IL-6 levels were independent predictors of the presence of cancer cachexia (p=0.0002, p<0.0001, respectively; univariate logistic regression: p=0.0017, p=0.0005, respectively; multivariate logistic regression). However, the best objective, quantifiable measure of anorexia/cachexia is weight loss. Serum MIC-1 levels were significantly associated with the degree of prostate cancer associated weight loss (Fig. 4a; p=0.0002, r=0.4899; linear regression), while there was no such relationship for serum IL-6 (p=0.6303; linear regression) (Figure 9b).

These results also raised the possibility that serum MIC-1 estimation may be useful as test for the prediction of prostate cancer associated cachexia. To assess this possibility, the current best serum marker (IL-6) was compared with serum MIC-1 level present upon diagnosis of cancer by receiver operator curve (ROC) analysis. Serum MIC-1 levels significantly out performed IL-6 as a predictor of cancer associated weight loss (Area Under the Curve (AUC) 0.6829 Vs 0.3505; p=0.0046). To put the diagnostic capability of serum MIC-1 for cancer cachexia in context, the ROC of the most widely used serum marker for the diagnosis of prostate cancer, PSA, has an AUC of 0.678 (95% confidence interval, 0.666-0.689)²⁴, almost identical characteristics to that of MIC-1. Consequently, serum MIC-1 measurement should prove useful for the prediction of cancer cachexia associated with prostate cancer and also identify those patients who might benefit from any future therapy designed to reduce serum levels of MIC-1.

### Serum MIC-1 levels are related to BMI in patients with chronic renal failure

Chronic renal failure, like advanced cancer may also be associated with weight loss and cachexia. Markers of this process such as anorexia, weight loss and BMI are strong predictors of mortality in end stage renal failure³⁰. As BMI is such strong predictor of mortality, and elevated serum MIC-1 levels were associated with similar changes in animals, the relationship between serum MIC-1 level and BMI in end stage renal failure was investigated. To do this, serum samples were examined from a cohort of 381 patients with end stage renal failure, that had been not been assembled to study cachexia or other metabolic processes³¹.

Patients who died during the study period (up to 3 years) had significantly lower BMI (26.17±5.63; 266 (mean±SD; n), 23.15±4.92; 104: p<0.0001; unpaired t-test). A predialysis serum sample was obtained at study entry and MIC-1 serum level determined. Serum MIC-1 level was correlated with BMI such that increasing serum MIC-1 levels were associated with lower BMI (p=0.0003; r=0.189; linear regression).

### Transgenic mice overexpressing MIC-1 are smaller and eat less

To determine the effects that might occur with long-term administration of MIC-1, C57BL6 transgenic mice overexpressing MIC-1 under the control of the CSF-1 promoter (fmsMIC-1 mice) were examined. These mice had similar weights to their WT controls when measured within the first 48 hours after birth (Figure 10a). However, by 21 days of age both male and female fmsMIC-1 mice weighed 18% less than their sex matched WT littermates (Figure 10a), a difference that persisted into adulthood. The fmsMIC-1 mice ate less (Figure 10b) than controls in absolute terms, though this difference disappeared when food intake was corrected for mouse weight. It is likely that decreased food intake from birth may have led to the development of mice that were smaller than syngeneic littermates and as a consequence ate proportionately less. Like the mice acutely treated with MIC-1, male and female fmsMIC-1 mice had reduced fat in all measured depots (Figure 10c). Overall, the data from the transgenic mice overexpressing MIC-1 is broadly consistent with mice acutely treated with this cytokine.

### Systemically administered MIC-1 activates hypothalamic neurons

The major regulatory centres for appetite and weight control reside in the hypothalamus, especially in the area of the arcuate nucleus (ARC), which is thought to have a semi-permeable blood brain barrier, potentially allowing the direct interaction of blood borne mediators with this area of the brain²⁵. To determine whether MIC-1 was exerting its effect directly on the brain, mice were injected i.p. with recombinant hMIC-1, then one hour later sacrificed them to examine the brain expression pattern of the immediate early gene c-fos. This transcription factor is rapidly induced by a wide variety of stimuli and is a frequently used, reliable indicator of neuronal activation²⁶. There was significantly increased c-fos immunoreactivity in the ARC and paraventricular nucleus (PVN) of the hypothalamus after MIC-1 injection, indicating neurons in these nuclei as downstream mediators of MIC-1 action. Interestingly, the brainstem area postrema (AP) also showed a significant increase in the number of c-fos positive neurons (Table 1), suggesting a potential role for this nucleus in response to MIC-1 action. There was no change in the number of c-fos positive neurons in the nucleus tractus solitarus (NTS) or in any other areas of the hypothalamus or brainstem (data not shown).

**TABLE 1**

| **Nucleus** | **% c-fos nuclei (Control)** | **% c-fos nuclei (MIC-1)** | **P Value** |
|---|---|---|---|
| **ARC** | 100 ± 6.01 | 138 ± 7.3 | 0.0036 |
| **PVN** | 100 ± 9.9 | 168 ± 9.3 | 0.001 |
| **AP** | 100 ± 22.5 | 377 ± 22.9 | 0.0014 |

| | | | |
|---|---|---|---|
| The nuclear staining for c-fos was quantitated in various regions of the brain including ARC, PVN, AP and NTS and presented as means and standard deviations. | | | |

These findings suggest that the central effect of MIC-1 is directed at the ARC, the major centre for appetite control. Projections from the ARC reach other hypothalamic nuclei such as the PVN from which connections are formed with brain stem areas including the AP, by which they specifically modulate vagal sympathetic nervous system activity²⁷. However, direct MIC-1 activation of the AP cannot be excluded. The lack of alteration in c-fos activation in the NTS suggests that afferent signals originating from the periphery via the vagus nerve are not involved in mediating MIC-1 effects.

### Hypothalamus specific overexpression of MIC-1 induces weight loss

To further demonstrate that MIC-1 induced weight loss occurred via a hypothalamic mediated effect, MIC-1 expression was induced by direct unilateral injection of adeno-associated virus (AAV) into the arcuate nucleus of the hypothalamus. This had been engineered to express full length human MIC-1 under the control of a neuronal specific enolase promoter. Immunohistochemistry with antibodies specific for human MIC-1 demonstrated the expression of this protein by cells in the ARC (Figure 11). Mice injected in this way with MIC-1 expressing AAV rapidly lost weight but mice injected with empty control vector gained weight in a normal manner. This provides further strong evidence for a direct effect of MIC-1 on cells of the hypothalamus.

### Systemically administered MIC-1 acts on the ARC through the TGF-β RII receptor

TGF-β superfamily proteins like MIC-1, bind to and act through a highly conserved receptor superfamily consisting of four distinct type II (RII) and seven type I receptors (RI), each containing serine/threonine kinase domains. Upon ligand binding, two RII and two RI receptors assemble into a tetramer in which the RII transphosphorylates serine residues in the cytoplasmic domain of RI. This starts a signalling cascade frequently involving the conserved smad family of transcription factors. Many other signalling molecules are also activated, including erk 1/2, that has been previously linked to MIC-1 triggered receptor activation^{14,32}.

To determine the RII receptor preferred by MIC-1, immunohistochemistry was conducted employing various antibodies against different RII receptors. This demonstrated that only the TGF-β RII was co-localised with c-fos in the hypothalamus in mice injected with MIC-1, 30 minutes prior to sacrifice (data not shown). To determine whether the TGF-β RII receptor formed part of the MIC-1 binding receptor complex, blocking antibodies to this receptor were injected unilaterally into the hypothalamus. After 24 hours these mice were injected i.p. with 10µg of MIC-1. The mice were sacrificed 30 min later and hypothalamic c-fos expression was examined.

Immunohistochemical nuclear c-fos expression was blocked on the side injected with the TGF-β RII antibody, but not on the contra-lateral side. Importantly, injection of blocking antibodies directed at the RII of the closely related bone morphogenetic protein (BMP), or the leptin receptor did not result in inhibition of MIC-1 induced c-fos expression (data not shown). This clearly demonstrates that the TGF-β RII receptor must form part of the complex through which MIC-1 exerts its effect in the hypothalamus.

To probe the mechanisms by which MIC-1 induces downstream effects in the hypothalamus, further immunohistochemistry was undertaken on the hypothalamus of mice injected i.p. with 100µg of MIC-1, between 15 and 60 minutes prior to sacrifice. Antibodies were utilised which were specific for the phosphorylated forms of members of the smad signalling pathway: smad 2, smad 3 and smad 1,5,8. In each ease, the ARG of the untreated mice already demonstrated the presence of nuclear staining with all antibodies suggestive of constitutive activation of these pathways in the hypothalamus (data not shown). No substantial increase in smad staining in the MIC-1 treated mice could be detected, suggesting that these pathways are not utilised or possibly that the constitutive activation of these pathways masks any significant increase in smad phosphorylation. However, MIC-1 has been reported by several groups to directly induce phosphorylation of erk 1/2 ³². Activation of this molecule was therefore examined in the hypothalamus of mice injected with 100µg of MIC-1 at 10 to 60 minutes prior to sacrifice. After an i.p. injection of MIC-1, P-erk 1/2 staining was detected in the ARC, with maximal occurring at 20 minutes after MIC-1 injection. There was no activation of this pathway in control buffer injected mice. Taken together, these data indicate that MIC-1 mediates effects via activation of the erk 1/2 pathway.

### Systemically administered MIC-1 modulates ARC NPY and POMC expression and activates the transcription factor, stat3

To determine whether systemic injection of MIC-1 was activating ARC neuronal pathways known to be associated with appetite regulation, the expression of two major appetite regulators, NPY and POMC, was investigated. Using *in situ* hybridisation, it was noted that MIC-1 injection reduced the level of NPY mRNA expression in the ARC by 34% and increased the mRNA levels of POMC by 47%, consistent with a reduced feeding drive (Table 2).

**TABLE 2**

| **mRNA (ARC)** | **% Positive (Control)** | **% Positive (MIC-1)** | **P Value** |
|---|---|---|---|
| **NPY** | 100 ± 13.3 | 66 ± 6.1 | 0.0272 |
| **POMC** | 100 ± 7.6 | 147 ± 10.5 | 0.0061 |

| | | | |
|---|---|---|---|
| Expressing cells were quantitated in the ARC and the results presented as means and standard deviations. | | | |

This upregulation of the major POMC anorexigenic pathway and downregulation of the NPY orexigenic pathway, is a similar pattern to that observed in leptin treated mice (data not shown). Because of this similarity, the hypothalamus of MIC-1 treated mice was also probed with antibodies to phosphorylated stat3 (P-stat3), a central molecule of the leptin receptor signalling pathway (data not shown). This clearly indicated marked upregulation of P-stat3 staining neurons in the lateral ARC and venteromedial hypothalamus in MIC-1 treated mice. Interestingly, the pattern of P-stat3 staining in MIC-1 treated mice was different from that induced by leptin injection, with the majority of P-stat3 positive neurons in MIC-1 injected mice being present in the lateral ARC compared to the dorsal ARC for leptin injection. Thus, MIC-1 and leptin, whilst both activating the stat3 signalling pathway in the ARC, induced P-stat3 on different subsets of ARC neurons. These findings suggest that MIC-1 and leptin induce their effects via distinct pathways. In keeping with this, injection of MIC-1 into leptin receptor deficient *db*/*db* mice produced comparable P-stat3 staining in the ARC to that seen in WT MIC-1 injected mice, conclusively demonstrating that MIC-1 does not act through the leptin receptor.

To further identify the MIC-1-induced P-stat3 positive neurons, *in situ* hybridisation for NPY and immunohistochemistry for alpha melanocyte stimulating hormone (α-MSH), a biologically active processed product of POMC, was undertaken along with immunohistochemistry/immunofluorescence for P-stat3 (data not shown). This identified α-MSH and NPY neurons as the major class of targets for MIC-1 and indicated that reduced food intake leading to weight loss in this model is due to altered NPY and α-MSH production. Further, and consistent with the results of the c-fos activation, the majority of these P-stat3 / NPY and P-stat3/ α-MSH positive neurons represent a different subset of neurons to the ones activated by leptin thereby indicating a novel pathway through which MIC-1 regulates appetite.

### Direct injection of MIC-1 into ARC also activates stat3

To provide further evidence that MIC-1 acts directly on the ARC, direct unilateral injection of MIC-1, leptin or control buffer into the ARC was investigated. Twenty minutes after injection was completed, mice were sacrificed and brain sections stained for P-stat3. Whilst control injection had no effect, both leptin and MIC-1 clearly induced activation of P-stat3 in the ARC (data not shown). The pattern of staining was similar to that seen with the systemically administered mediators, with MIC-1 injection inducing the phosphorylation of stat3 in neurons in the lateral part of the ARC and the dorsal part of the ventromedial hypothalamic nucleus (VMHDM). In contrast, leptin injection activates the Stat3 pathway in neurons of the dorsal part of the ARC, the dorsal part of the VMHDM and lateral hypothalamic area (LHA) (data not shown). This further indicates that these two mediators act on different neuronal subsets in the hypothalamus.

### Discussion

Tumour overproduction of MIC-1 and the strong correlation of serum MIC-1 levels with weight loss in both animal models and patients with prostate cancer, indicates that MIC-1 is involved in the pathogenesis of cancer anorexia/cachexia and perhaps other cachectic conditions, such as those that are associated with cardiac and renal failure. While many studies describe elevated cytokine levels in groups of people with cancer anorexia/cachexia compared to those without this complication, no one has demonstrated a direct correlation of cytokine levels with the degree of weight loss that is evident for MIC-1. This relationship, as well as the strong body of mechanistic data provided herein indicating that MIC-1 acts on TGF-β RII receptors in hypothalamic neurones to decrease appetite, establishes that MIC-1 is an important cause of cancer related anorexia and weight loss. Additionally, reversal of anorexia/cachexia by antibodies to MIC-1 indicates the potential of using therapeutic antibodies (and other MIC-1 antagonists) to treat this serious disorder. Lastly, the results presented herein indicate that MIC-1 causes hypophagia and is able to directly influence the major hypothalamic regulators of food intake and energy homeostasis, NPY and POMC-derived α-MSH. Further, while MIC-1 induces the leptin-like effects of anorexia, weight loss and decrease in hypothalamic NPY and increase in POMC expression, there are differences in the subset of NPY and POMC neurons activated by MIC-1 compared to leptin. This indicates a more diverse functional network of neurons regulating different aspects of energy homeostasis and, taken together, demonstrates that MIC-1 is a central regulator of appetite and weight and offers considerable potential, along with its receptor complex, as the target for the treatment of both cancer anorexia/cachexia and obesity.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

All publications mentioned in this specification are herein incorporated by reference. Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia or elsewhere before the priority date of each claim of this application.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

### REFERENCES

1. Tisdale, MJ. Cachexia in cancer patients. Nat Rev Cancer, 2:862-871 (2002).
2. Huhmann, MB. Cunningham, RS. Importance of nutritional screening in treatment of cancer-related weight loss. Lancet Oncol, 6:334-343 (2005).
3. Marks, DL. Ling, N. Cone, RD. Role of the central melanocortin system in cachexia. Cancer Res, 61:1432-1438(2001).
4. Rubin, H. Cancer cachexia: its correlations and causes. Proc Natl Acad Sci, 100:5384-5389 (2003).
5. Rall, LC. Roubenoff, R. Rheumatoid cachexia: metabolic abnormalities, mechanisms and interventions. Rheumatology 43:1219-1223 (2004).
6. Anker, SD. Sharma, R. The syndrome of cardiac cachexia. Int J Cardiol 85:51-66 (2002).
7. Bootcov, MR. et al. MIC-1, a novel macrophage inhibitory cytokine, is a divergent member of the TGF-β superfamily cluster. Proc Natl Acad Sci 94:11514- 11519 (1997).
8. Welsh, JB. et al. Large-Scale Delineation of Secreted Protein Biomarkers Overexpressed in Cancer Tissue and Serum. Proc Natl Acad Sci 100:3410-3415 (2003).
9. Buckhaults, P. et al. Secreted and cell surface genes expressed in benign and malignant colorectal tumours. Cancer Res 61:6996-7001 (2001).
10. Koopmann,J. et al. Serum Macrophage Inhibitory Cytokine 1 as a Marker of Pancreatic and other Periampullary Cancers. Clin Cancer Res 10:2386-2392 (2004).
11. Bauskin, AR. et al. The propetide mediates formation of stromal stores of proMIC-1: Role in determining prostate cancer outcome. Cancer Res 65:2330-2336 (2005).
12. Hsiao, EC. et al. Characterization of growth-differentiation factor 15, a transforming growth factor beta superfamily member induced following liver injury. Mol Cell Biol 20:3742-51 (2000).
13. Schober. A. et al. Expression of growth differentiation factor-15/ macrophage inhibitory cytokine-1 (GDF-15/MIC-1) in the perinatal, adult, and injured rat brain. J Comp Neurol 439:32-45 (2001).
14. Bauskin, AR. et al. Role of MIC-1 in Tumourigenesis and Diagnosis of Cancer. Cancer Res 66:4983-4986 (2006).
15. Fairlie, WD. et al. Epitope mapping of the Transforming Growth Factor-β superfamily protein, Macrophage Inhibitory Cytokine-1 (MIC-1): Identification of at least five distinct epitope specificities. Biochemistry 40:65-73 (2001).
16. Brown, DA. et al. Concentration in plasma of macrophage inhibitory cytokine-1 and the risk of cardiovascular events in women. Lancet 359:2159-2163 (2002).
17. Challet, E. et al. Involvement of corticosterone in the fasting-induced rise in protein utilization and locomotor activity. Pharmacol Biochem Behav 50:405-412 (1995).
18. Kamel, HK. Maas, D. Duthie, EH. Role of hormones in the pathogenesis and management of sarcopenia. Drugs Aging 19:865-877 (2002).
19. Rosen, CJ. et al. Congenic mice with low serum IGF-I have increased body fat, reduced bone mineral density, and an altered osteoblast differentiation program. Bone 35:1046-1058 (2004).
20. Park, S. Sohn, S. Kineman, RD. Fasting-induced changes in the hypothalamic-pituitary-GH axis in the absence of GH expression: lessons from the spontaneous dwarf rat. JEndocrinol 180:369-378 (2004).
21. Lewitt, MS et al. Responses of insulin-like growth factor (IGF)-I and IGF-binding proteins to nutritional status in peroxisome proliferator-activated receptor-alpha knockout mice. Growth Horm IGF Res 11:303-313 (2001).
22. Friedl, KE. et al. Endocrine markers of semistarvation in healthy lean men in a multistressor environment. J Appl Physiol 88:1820-1830 (2000).
23. Pfitzenmaier, J. et al. Elevation of cytokine levels in cachectic patients with prostate carcinoma. Cancer 97:1211-1216 (2003*).*
24. Thompson, IM. et al. Operating characteristics of prostate-specific antigen in men with an initial PSA level of 3.0 ng/ml or lower. JAMA 294:66-70 (2005).
25. Naylor, JL. et al. Further evidence that the blood/brain barrier impedes paraquat entry into the brain. Human & Experimental Toxicology 14:587-594 (1995).
26. Karl, T. et al. Y1 receptors regulate aggressive behavior by modulating serotonin pathways. Proc. Natl. Acad. Sci 101:12742-12747 (2004).
27. Bouret, S. et al. Formation of Projection Pathways from the Arcuate Nucleus of the Hypothalamus to Hypothalamic Regions Implicated in the Neural Control of Feeding Behavior in Mice. J NeuroSci 24:2797-2805 (2004).
28. Sainsbury, A. Schwarzer, C. Couzens, M. Herzog, H. Y2 receptor deletion attenuates the type 2 diabetic syndrome of ob/ob mice. Diabetes 51:3420-3427 (2002).
29. Paxinos, G. Franklin, KBJ. The mouse brain in sterotaxic coordinates. 2001. Academic press. San Diego, USA.
30. Kovesdy, C. P., Anderson, J. E. & Kalantar-Zadeh, K. Inverse association between lipid levels and mortality in men with chronic kidney disease who are not yet on dialysis: effects of case mix and the malnutrition-inflammation-cachexia syndrome. J Am Soc Nephrol 18:304-311 (2007).
31. Apple, FS., Murakami, MM., Pearce, LA. & Herzog, CA. Predictive value of cardiac troponin I and T for subsequent death in end-stage renal disease. Circulation 106:2941-2945 (2002).
32. Lee, DH. et al. Macrophage inhibitory cytokine-1 induces the invasiveness of gastric cancer cells by up-regulating the urokinase-type plasminogen activator system. Cancer Res 63(15):4648-4655 (2003).
33. Moore, AG. et al. TGF-β superfamily cytokine MIC-1 is present in high concentrations in the serum of pregnant women. J Clin Endocrinol Metab 85:4781-4788 (2000).
34. Wollman, W. et al. The macrophage inhibitory cytokine integrates AKT/PKB and MAP kinase signaling pathways in breast cancer cells. Carcinogenesis 26(5):900-907 (2005).
35. DaCosta Byfield, S. et al. SB-505124 is a selective inhibitor of transforming growth factor-beta type I receptors, ALK4, ALK5 and ALK7. Mol Pharmacol 65:744-752 (2004).
36. Bonniaud, P. et al. Progressive transforming growth factor β1-induced lung fibrosis is blocked by an orally active ALK5 kinase inhibitor. Am J Respir Crit Care Med 171:889-898 (2005).
37. de Gouville, AC. et al. Inhibition of TGF-beta signaling by an ALK5 inhibitor protects rats from dimethylnitrosamine-induced liver fibrosis. Br J Pharmacol 145:166-177 (2005).
38. Konrad, L et al. Alternative splicing of TGF-betas and their high-affinity receptors TβRI, TβRII and TβRIII (betaglycan) reveal new variants in human prostatic cells. BMC Genomics 8:318 (2007).

### Aspects of the invention may also be described by the following numbered paragraphs:-

1. A method of modulating the activity of macrophage inhibitory cytokine-1 (MIC-1) in a subject, said method comprising administering to said subject an effective amount of an agent which interacts and modulates the activity of a MIC-1 receptor complex comprising an RII receptor for transforming growth factor-β (TGF-β), optionally in admixture with a pharmacologically-acceptable carrier and/or excipient.
2. The method of paragraph 1, wherein the agent interacts and modulates the activity of MIC-1 receptor complex.
3. The method of paragraph 2, wherein the agent inhibits the activity of MIC-1 to bring about an increase in appetite and/or body weight of a subject.
4. The method of paragraph 3, when used for treating anorexia/cachexia associated with MIC-1 overexpression.
5. The method of any one of paragraphs 2 to 4, wherein the agent interacts with said TGF-β RII receptor.
6. The method of paragraph 5, wherein the TGF-β RII receptor is a hypothalamic TGF-β RII receptor.
7. The method of any one of paragraphs 2 to 4, wherein the agent is selected from the group consisting of ALK5 inhibitors, inhibitory peptide fragments and mimetics of MIC-1, anti-TGF-β RII antibodies and fragments thereof, and anti-RI antibodies and fragments thereof.
8. The method of paragraph 2, wherein the agent enhances the activity of MIC-1 to bring about a decrease in appetite and/or body weight of a subject.
9. The method of paragraph 8, when used for treating obesity.
10. The method of paragraph 8 or 9, wherein the agent interacts with said TGF-β RII receptor.
11. The method of paragraph 10, wherein the TGF-β RII receptor is a hypothalamic TGF-β RII receptor.
12. The method of any one of paragraphs 8 to 11, wherein the agent is selected from the group consisting of stimulatory peptide fragments and mimetics of MIC-1.
13. The method of paragraph 1, wherein the agent modulates the amount of MIC-1 receptor complex in the subject.
14. The method of paragraph 13, wherein the agent decreases the amount of MIC-1 receptor complex in the subject.
15. The method of paragraph 14, wherein the agent is selected from the group consisting of catalytic and inhibitory oligonucleotide molecules targeted against the gene(s) encoding the MIC-1 receptor complex, and inhibitors of MIC-1 receptor complex transcription or translation.
16. The method of paragraph 14 or 15, wherein the agent decreases the amount of MIC-1 receptor complex by decreasing the amount of endogenous TGF-β RII receptors.
17. The method of paragraph 16, wherein the TGF-β RII receptors are hypothalamic TGF-β RII receptors.
18. The method of any one of paragraphs 13 to 17, when used for treating anorexia/cachexia associated with MIC-1 overexpression.
19. The method of paragraph 4 or 18, wherein the MIC-1 overexpression is due to cancer.
20. The method of paragraph 4 or 18, wherein the MIC-1 overexpression is due to chronic renal failure.
21. A method for screening an agent for capability to modulate the activity of macrophage inhibitory cytokine (MIC-1), said method comprising the steps of:
   (i) contacting said agent with a MIC-1 receptor complex comprising the RII receptor for transforming growth factor-β (TGF-β), or a monomer thereof, and
   (ii) detecting any change in the activity of said MIC-1 receptor complex or monomer thereof.
22. The method of paragraph 21 wherein said MIC-1 receptor complex or monomer thereof is provided on the surface of cultured cells.
23. The method of paragraph 21 or 22, wherein changes in the activity of the MIC-1 receptor complex or monomer thereof may be detected by detecting the activation or lack of activation, of the erk 1/2, P-stat3 signalling molecules or smad and/or akt signalling pathways.
24. The method of any one of paragraphs 21 to 23, wherein the TGF-β RII receptor is the hypothalamic TGF-β RII receptor.
25. A novel agent identified by the method of any one of paragraphs 21 to 24.

## Claims

1. An agent which binds to and blocks a TGF-β RII receptor, optionally in admixture with a pharmacologically-acceptable carrier and/or excipient, for use in a method of inhibiting the activity of MIC-1 in a subject.

2. The agent of claim 1, wherein the subject is suffering from anorexia or cachexia associated with MIC-1 overexpression.

3. The agent of claim 1 or 2, wherein the agent is selected from the group consisting of an anti-TGF-β RII antibody and antibody fragments thereof, and inhibitory peptide fragments and mimetics of MIC-1.

4. The agent of any one of claims 1 to 3, wherein the agent is an anti-TGF-β RII antibody or antibody fragment thereof.

5. The agent of claim 4, wherein the anti-TGF-β RII antibody is a humanized antibody.

6. The agent of claim 1, wherein the agent interacts with a hypothalamic TGF-β RII receptor.

7. The agent of any one of claims 1 to 6, wherein the TGF-β RII receptor is a member of a MIC-1 receptor complex.

8. An agent which binds to and blocks a TGF-β RII receptor, optionally in admixture with a pharmacologically-acceptable carrier and/or excipient, for use in a method of inhibiting weight loss in a subject.

9. The agent of claim 8, wherein the weight loss observed in the subject is associated with MIC-1 overexpression.

10. The agent of claim 8, wherein the subject is suffering from anorexia or cachexia associated with MIC-1 overexpression.

11. The agent of any one of claims 8 to 10, wherein the agent is selected from the group consisting of an anti-TGF-β RII antibody and antibody fragments thereof, and inhibitory peptide fragments and mimetics of MIC-1.

12. The agent of any one of claims 8 to 11, wherein the agent is an anti-TGF-β RII antibody or antibody fragment thereof.

13. The agent of claim 12, wherein the anti-TGF-β RII antibody is a humanized antibody.

14. The agent of claim 8, wherein the agent interacts with a hypothalamic TGF-β RII receptor.

15. The agent of any one of claims 8 to 14, wherein the TGF-β RII receptor is a member of a MIC-1 receptor complex.


SEQUENCE LISTING

<110>  St Vincent's Hospital Sydney Limited

<120>  AGENTS AND METHODS FOR MODULATING MACROPHAGE INHIBITORY CYTOKINE
       (MIC-1) ACTIVITY

<130>  P039511EPA

<140>  08782955.2
<141>  2008-08-18

<150>  AU2007904412
<151>  2007-08-16

<160>  3

<170>  PatentIn version 3.5

<210>  1
<211>  42
<212>  DNA
<213>  Mus musculus

<220>
<221>  misc_feature
<223>  NPY

<400>  1
gagggtcagt ccacacagcc ccattcgctt gttacctagc at                          42

<210>  2
<211>  40
<212>  DNA
<213>  Mus musculus

<220>
<221>  misc_feature
<223>  POMC

<400>  2
tggctgctct ccaggcacca gctccacaca tctatggagg                             40

<210>  3
<211>  45
<212>  DNA
<213>  Mus musculus

<220>
<221>  misc_feature
<223>  GHRH

<400>  3
gcttgtcctc tgtccacatg ctgtcttcct ggcggctgag cctgg                       45